(19) Europäisches Patentamt
European Patent Office
Office européen des brevets

(11) Veröffentlichungsnummer : **0 442 400 B1**

(12) **EUROPÄISCHE PATENTSCHRIFT**

(45) Veröffentlichungstag der Patentschrift : **15.06.94 Patentblatt 94/24**

(51) Int. Cl.$^5$ : **C07C 19/08,** C07C 17/20, C07C 41/22, C07C 43/12

(21) Anmeldenummer : **91101828.1**

(22) Anmeldetag : **09.02.91**

# (54) Verfahren zur Herstellung von weitgehend fluorierten aliphatischen Kohlenwasserstoffen, die 1 oder 2 Brom- oder Chloratome im Molekül aufweisen.

(30) Priorität : **16.02.90 DE 4004783**

(43) Veröffentlichungstag der Anmeldung : **21.08.91 Patentblatt 91/34**

(45) Bekanntmachung des Hinweises auf die Patenterteilung : **15.06.94 Patentblatt 94/24**

(84) Benannte Vertragsstaaten : **BE DE FR GB IT NL**

(56) Entgegenhaltungen :
**EP-A- 0 194 781**
**EP-A- 0 298 870**

(56) Entgegenhaltungen :
**CHEMICAL ABSTRACTS, Band 104, Nr. 11, 17. MUrz 1986, Columbus, Ohio,USA;FURUTAKA et al. "Perfluoroalkyl bromides" Seite 619, Zusammenfassung-Nr.88 106p & JP-A-60184033**
**CHEMICAL ABSTRACTS, Band 97, Nr. 3, 19. Juli 1982, Columbus, Ohio, USA BLANCOUet al. "Reactivity of perlfluoroiodoalkane with halo derivatives indissociating aprotic solvents (DMSO and DMF)" Seite 660, Zusammenfassung-Nr.23243y**

(73) Patentinhaber : **HOECHST AKTIENGESELLSCHAFT**
**D-65926 Frankfurt (DE)**

(72) Erfinder : **von Werner, Konrad, Dr.**
**Wehrstrasse 6**
**W-8268 Garching (DE)**

**EP 0 442 400 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Herstellung von weitgehend fluorierten aliphatischen Kohlenwasserstoffen, die 1 oder 2 Brom- oder Chloratome im Molekül aufweisen aus den entsprechenden Iod enthaltenden Verbindungen.

Es ist aus US-PS 2,678,953 bekannt, Perfluoralkylbromide aus den trockenen Metallsalzen von Perfluoralkylcarbonsäuren durch Umsetzung mit Brom zu gewinnen, wobei die Anwendung von sichtbarem Licht den Umsatz steigert. Im einzigen Beispiel wird das bevorzugte Silbersalz verwendet. Dieses Verfahren ist aufwendig, da erst die Perfluoralkylcarbonsäure erzeugt werden muß, beispielsweise durch die bekannte Umsetzung von Perfluoralkyliodid mit $SO_3$ oder rauchender Schwefelsäure. Die Säure muß dann in das Metallsalz umgewandelt und dieses getrocknet werden. Ferner erfordert die Umsetzung mit dem giftigen und ätzenden Brom besondere Vorsicht und einen erhöhten apparativen Aufwand (Sicherheitsvorkehrungen, Korrosion). Außerdem geht bei der Reaktionskette eine $CF_2$-Gruppe des Perfluoralkyliodids verloren.

Es ist ferner aus US-PS 2,875,253 bekannt, einen mit Fluor, Brom und gegebenenfalls mit Chlor substituierten, niedrigen Kohlenwasserstoff als Telogen mit einem Fluor enthaltenden Olefin, das zusätzlich Chloratome enthalten kann, in Gegenwart eines peroxidischen Polymerisations-Promotors zu telomerisieren. Als mögliche Telogene sind unter anderem $CF_3Br$, $CF_2BrCl$, $CF_2Br_2$, $C_2F_5Br$, $C_2F_4BrCl$, $C_3F_6BrH$ und $C_3F_6Br_2$ genannt, unter einer Vielzahl möglicher Fluor enthaltender Olefine Tetrafluorethylen. Die Umsetzung dieser Verbindungen müßte zu weitgehend fluorierten Alkylbromiden im Sinne der vorliegenden Erfindung führen, jedoch ist kein Beispiel vorhanden, aus dem zu entnehmen wäre, unter welchen genauen Bedingungen und mit welchem Erfolg die Umsetzung mit Tetrafluorethylen durchführbar ist. In den Beispielen wird als Fluor enthaltendes Olefin immer $CF_2$=CFCl eingesetzt.

In einem Aufsatz von Long, Higgins, Mattrey, Mitten und Multer über Radiopake Fluorkohlenwasserstoffe (R.E. Banks, "Preparation, Properties and Industrial Applications of Organofluorine Compounds", 1982, Ellis Horwood Ltd. Publishers/Chichester, Seiten 139 bis 156) ist am Ende der Ausführungen (Seite 154 unten) erwähnt, daß das für die Untersuchungen verwendete Perfluor-n-hexylbromid beziehungsweise Perfluor-isoheptylbromid durch thermische Bromierung der entsprechenden Perfluoralkyliodide mit elementarem Brom erzeugt wurde, doch fehlen nähere Angaben. zweifellos steigen die weiter oben bereits erwähnten apparativen Schwierigkeiten bei Anwendung von Brom, wenn thermisch, das heißt bei höheren Temperaturen, mit diesem aggressiven Stoff gearbeitet wird.

R. N. Haszeldine, J. Chem. Soc., 1953, Seiten 3 761 bis 3 768 sowie Huang Bingnan und Huang Weiyuan, Shanghai Inst. Org. Chem. Acad. Sinica, Huaxue Xuebao 42, Seiten 1 106 bis 1 108 (C.A. 102; 78312x), 1984 beschreiben die photochemische Bromierung von Perfluoralkyliodiden [Beispiele: $R_fI$ oder $Cl(CF_2)_4I$] mit Brom unter UV-Strahlung. Nach 168 beziehungsweise 50 Stunden Umsetzung wird $R_fBr$ oder $Cl(CF_2)_4Br$ in sehr guter Ausbeute erhalten. Allerdings ist auch dieses Verfahren apparativ und energetisch aufwendig.

Ferner ist aus JP-Kokai 60-184033-A2, 1985 (C.A. 104; 88106p) bekannt, $C_nF_{2n+1}Br$ (n = 6 bis 11) durch Umsetzung von $C_nF_{2n+1}I$ mit Brom in Gegenwart einer Radikale erzeugenden Verbindung (zum Beispiel Azodiisobutyronitril) zu erzeugen. Es wird so $C_6F_{13}Br$ mit 40 % Ausbeute gewonnen.

Schließlich ist es aus EP 194 781 bekannt, α-ω-Dichlor- oder -Dibromperfluoralkylen-Verbindungen durch Umsetzung der entsprechenden Iodverbindungen, die durch Telomerisation mit Tetrafluorethylen gewonnen wurden, mit elementarem Chlor oder Brom bei Temperaturen bis 180 °C zu erzeugen. Die Anwendung der elementaren, agressiven Halogene bei höheren Temperaturen erfordert auch bei den beiden zuletzt genannten Verfahren besondere apparative Vorkehrungen.

Es wurde nun gefunden, daß das zuletzt beschriebene Verfahren verbessert werden kann, wenn bestimmte Metallkomplex-Verbindungen eingesetzt werden, wobei nicht nur weitgehend fluorierte Alkylbromide, sondern auch die entsprechenden Chloride mit deutlich günstigeren Ausbeuten erzeugt werden können.

Das neue Verfahren zur Herstellung von weitgehend fluorierten aliphatischen Kohlenwasserstoffen, die 1 oder 2 Brom- oder Chloratome im Molekül aufweisen aus den entsprechenden weitgehend fluorierten aliphatischen Kohlenwasserstoffen, die 1 oder 2 primär oder sekundär gebundene Iodatome im Molekül aufweisen, durch Umsetzung von 1 mol gebundenem Iodatom in der Iodverbindung mit 1 bis 4 mol Bromid- oder Chloridionen, die als Salze mit mindestens einem der folgenden Kationen: Li, Na, K, Mg, Ca, Mn oder substituiertes Ammonium vorliegen, in mindestens einem dipolaren, aprotischen Lösungsmittel bei 20 bis 140 °C unter normalem Atmosphärendruck oder unter dem autogenen Druck der Reaktionsmischung, ist dadurch gekennzeichnet, daß die Umsetzung durchgeführt wird in Gegenwart einer Metall-Komplex-Verbindung, die als Zentralatom Chrom, Nickel, Kobalt oder Rhodium enthält und die je Zentralatom bei der Herstellung der weitgehend fluorierten aliphatischen Bromide 1, 2 oder 3 Bromatome, bei der Herstellung der weitgehend fluorierten aliphatischen Chloride 1, 2 oder 3 Chloratome sowie als weitere Liganden mindestens einen der folgenden Formeln enthält:

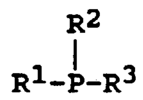

worin bedeuten: $R^1$, $R^2$, $R^3$, gleich oder verschieden, Alkyl mit 1 bis 8 C-Atomen, Alkoxy mit 1 bis 8 C-Atomen, Phenyl oder Phenoxy, das mit Alkyl-, Alkoxy-, Phenyl- oder Phenoxy-Gruppen substituiert sein kann und das 6 bis 14 C-Atome aufweist, oder

$$(Phenyl)_2P\text{-}R^4\text{-}P(Phenyl)_2$$

worin $R^4$ einen Alkylenrest mit 1 bis 6 C-Atomen bedeutet.

Als Ausgangsstoffe für das erfindungsgemäße Verfahren sind Verbindungen folgender Formel geeignet

$$X\text{-}R\text{-}I \qquad (I)$$

in der bedeuten X = H oder ein Halogenatom und R einen geradkettigen oder verzweigten perfluorierten Alkylenrest, der 1 bis 16 C-Atome enthält, wenn X = H, F oder Cl ist, und der 3 bis 16 C-Atome enthält, wenn X = Br oder I ist, und der ferner auf je mindestens 4 C-Atome ein Ethersauerstoffatom enthalten kann. Geeignete Verbindungen sind beispielsweise

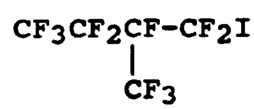

$$CF_3CF_2CF_2OCF_2CF_2I$$

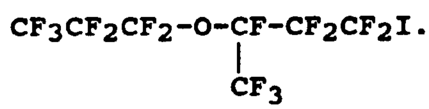

vorzugsweise werden solche Verbindungen der Formel (I) eingesetzt, die keine Ethersauerstoffatome enthalten, ferner sind bevorzugt solche Verbindungen, in denen X = I ist und insbesondere solche, in denen X F bedeutet und R ein perfluorierter Alkylenrest ist, der entweder geradkettig oder in $\omega$-Stellung zum Iodatom durch eine Methylgruppe verzweigt ist. Verbindungen der Formel (I), in denen der Rest R mehr als 16 C-Atome enthält, ergeben im allgemeinen längere Reaktionszeiten und häufig schlechtere Ausbeuten, sie sind auch in der Regel weniger gut verwendbar. Wegen ihrer guten Anwendbarkeit sind Verbindungen der Formel (I) bevorzugt, in denen R ein Alkylenrest mit 2 bis 12 und insbesondere mit 6 bis 8 C-Atomen ist. Anstelle der Verbindungen der Formel (I) können auch solche eingesetzt werden, die anstatt des oder der primären Iodatome(s) sekundär gebundene Iodatome enthalten. Hierbei ist unter einem primär gebundenen Iodatom ein solches zu verstehen, das mit einem Kohlenstoffatom verbunden ist, das seinerseits entweder eine oder gar keine Bindung zu einem weiteren Kohlenstoffatom aufweist, während ein sekundär gebundenes Iodatom ein solches ist, das mit einem Kohlenstoffatom verbunden ist, welches seinerseits mit zwei weiteren Kohlenstoffatomen je eine Einfachbindung hat. Es können auch Mischungen von Verbindungen der Formel (I) eingesetzt werden, die unterschiedliche Substituenten X und/oder unterschiedliche perfluorierte Alkylenreste R enthalten sowie auch Mischungen von Verbindungen mit primär gebundenen und sekundär gebundenen Iodatomen.

Erfindungsgemäß wird 1 mol gebundenes Iodatom der Verbindung der Formel (I) oder einer entsprechenden Verbindung mit sekundär gebundenen Iodatomen mit 1 bis 4 mol Bromidionen oder Chloridionen, die als Salze mit verschiedenen Kationen vorliegen können, umgesetzt. Werden je 1 mol Iodatom in der Verbindung der Formel (I) beziehungsweise einer entsprechenden Verbindung mit sekundär gebundenen Iodatomen weniger als 1 mol Bromidion oder Chloridion verwendet, so werden schlechtere Ausbeuten erhalten. Prinzipiell können über 4 mol Bromid- oder Chloridionen je 1 mol Iodatom eingesetzt werden, jedoch wird im allgemeinen hierdurch keine Ausbeuteverbesserung beobachtet, so daß dies ein unnötiger Aufwand ist. Vorzugsweise werden je 1 mol Iodatom in der Verbindung der Formel (I) oder einer entsprechenden Verbindung mit sekundär gebundenen Iodatomen 1,2 bis 2 mol Bromid- oder Chloridionen verwendet.

Die Bromid- oder Chloridionen können als Salz des substituierten Ammoniums vorliegen; als Substituenten am Stickstoff des Ammoniums kommen beispielsweise in Frage Alkylgruppen mit 1 bis 20 C-Atomen und/oder Hydroxyalkylgruppen mit 2 bis 4 C-Atomen, wobei die Gesamtzahl der C-Atome im substituierten Ammonium 40 nicht übersteigen sollte. Ferner können die Bromid- oder Chloridionen als Salz mit mindestens einem der folgenden Kationen, Natrium, Kalium, Magnesium oder Calcium, vorliegen. Vorzugsweise werden Bromide oder Chloride des Mangans oder des Tetraalkylammoniums eingesetzt. Besonders gute Ergebnisse werden erhalten, wenn die Bromid- oder Chloridionen zumindest teilweise als Lithiumsalze vorliegen. Es können auch Mischungen von Salzen, die entweder das Bromidion oder das Chloridion, jedoch verschiedene Kationen aufweisen, eingesetzt werden.

Erfindungsgemäß wird die Umsetzung der Verbindungen der Formel (I) oder entsprechender Verbindungen mit sekundären Iodatomen mit den Bromid- oder Chloridionen enthaltenden Salzen in Gegenwart einer Metall-Komplex-Verbindung, die als Zentralatom Chrom, Nickel, Kobalt oder Rhodium enthält, durchgeführt, die je Zentralatom bei Herstellung von weitgehend fluorierten aliphatischen Bromiden 1, 2 oder 3 Bromatome und bei Herstellung der weitgehend fluorierten aliphatischen Chloride 1, 2 oder 3 Chloratome enthält. Vorzugsweise wird eine Metall-Komplex-Verbindung mit Nickel als Zentralatom, die 2 Bromatome beziehungsweise 2 Chloratome aufweist, verwendet.

Die Metall-Komplex-Verbindung soll als weitere Liganden mindestens einen der folgenden Formeln enthalten

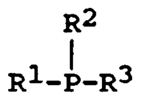

(II)

worin bedeuten: $R^1$, $R^2$, $R^3$, gleich oder verschieden, Alkyl mit 1 bis 8 C-Atomen, Alkoxy mit 1 bis 8 C-Atomen, Phenyl oder Phenoxy, das mit Alkyl-, Alkoxy-, Phenyl- oder Phenoxy-Gruppen substituiert sein kann und das 6 bis 14 C-Atome aufweist; oder

$(Phenyl)_2P-R^4-P(Phenyl)_2$ (III)

worin $R^4$ einen Alkylenrest mit 1 bis 6 C-Atomen bedeutet. Wegen ihrer guten Wirkung und günstigen Zugänglichkeit sind solche Metall-Komplex-Verbindungen bevorzugt, die mindestens einen Liganden der Formel (II) tragen, in der $R^1$, $R^2$ und $R^3$ gleich sind und bedeuten: einen Alkylrest mit 4 bis 8 C-Atomen, einen Alkoxyrest mit 2 bis 4 C-Atomen, einen Phenyl- oder einen Phenoxyrest. Bevorzugt sind auch solche Metall-Komplex-Verbindungen, die mindestens einen Liganden der Formel (III) tragen, in der $R^4$ einen Alkylenrest mit 2 bis 4 C-Atomen bedeutet.

Außer den Liganden der Formeln (II) oder (III) kann die Metall-Komplex-Verbindung noch weitere für solche Verbindungen bekannte Liganden enthalten, beispielsweise CO, NO, $NZ_3$, $AsZ_3$, $SbZ_3$, worin Z ein Alkylrest mit 1 bis 6 C-Atomen oder einen Aryl- oder Alkylarylrest mit 6 bis 12 C-Atomen bedeutet, sowie heterocyclische Verbindungen, die Stickstoffatome im Ring aufweisen, wie Pyridin. Die Metall-Komplex-Verbindung kann auch als Anion vorliegen, das mit einem geeigneten Kation, beispielsweise einem Alkali- oder Erdalkalimetall, eine salzartige Verbindung bildet.

Beispiele für geeignete Metall-Komplex-Verbindungen sind: $[(C_4H_9)_3P]_2NiBr_2$; $[(C_2H_5O)_3P]_2NiBr_2$; $[(Phenyl)_3P]_2NiCl_2$; $[(Phenyl)_3P]_2NiBr_2$; $[(Phenyl)_3P]_2Ni_2Br_4$; $Li[(Phenyl)_3PNiBr_3]$; $[(p\text{-}Tolyl)_3P]_2NiCl_2$; $[(Phenyl)_3P]_2Ni(NO)Br$; $[(Phenyl)_2P-(CH_2)_2-P(Phenyl)_2]NiCl_2$; $[(Phenyl)_2P-(CH_2)_4-P(Phenyl)_2]NiBr_2$; $[(Phenyloxy)_3P]_2NiBr_2$; $[(Phenyl)_3P]_2CoCl_2$; $[(p\text{-}Tolyl)_3P]_2CoBr_2$; $[(Phenyl)_2P-(CH_2)_2-P(Phenyl)_2]CoBr_2$; $[(Phenyl)_3P]_2Rh(CO)Br$; $[(Phenyl)_3P]_4CrBr_2$.

Ferner sind auch heterogene Metall-Komplex-Verbindungen geeignet, bei denen der Metall-Komplex in einer Polymerkette enthalten ist, wie zum Beispiel

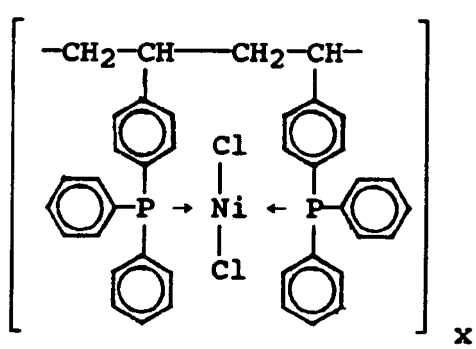

Die Herstellung der genannten Metall-Komplex-Verbindungen ist bekannt, siehe beispielsweise Gmelins Handbuch der anorganischen Chemie, 8. Auflage, Verlag Chemie/Weinheim, BR Deutschland, Band 52, Teil

C, Seite 19; Band 57, Teil C, Lieferung 2, Seite 1031 ff. und Band 64 (Rh), Supplement Volume B1, Seite 92. Die Metall-Komplex-Verbindungen können als solche vor ihrem Einsatz hergestellt werden oder auch, in manchen Fällen vorteilhaft, in situ durch Zugabe der Ausgangsstoffe in die Reaktionsmischung, die bereits eine Verbindung der Formel (I) oder eine entsprechende Verbindung mit sekundär gebundenen Iodatomen und ein Bromid- oder Chloridsalz enthält.

Je 1 mol gebundenem Iodatom in der Iodverbindung werden 0,001 bis 1 mol Metall-Komplex-Verbindung verwendet. Unter 0,001 mol Metall-Komplex-Verbindung wird im allgemeinen nicht mehr die erwünschte Ausbeuteerhöhung festgestellt, über 1 mol tritt im Rahmen des erfindungsgemäßen Verfahrens im allgemeinen keine Verbesserung des erfinderischen Effektes mehr ein. Es können jedoch auf 1 mol Iodatome in der Verbindung der Formel (I) oder einer entsprechenden Verbindung mit sekundär gebundenen Iodatomen auch über 1 mol, beispielsweise 1,1 bis 2 mol Metall-Komplex-Verbindung, verwendet werden, wobei die Zugabe von Bromidionen beziehungsweise Chloridionen, die als Salze vorliegen, nicht mehr erforderlich ist. Jedoch ist dieses Verfahren relativ aufwendig, so daß es, wenn überhaupt, nur in Sonderfällen lohnend anzuwenden ist. Vorzugsweise werden 0,02 bis 0,2 mol der Metall-Komplex-Verbindung je 1 mol Iodatome in der Iod enthaltenden Verbindung angewendet.

Die erfindungsgemäße Umsetzung wird in einem dipolaren, aprotischen Lösungsmittel durchgeführt. Hierbei werden auf 1 g der Verbindung der Formel (I) oder einer entsprechenden sekundär gebundene Iodatome enthaltenden Verbindung 0,5 bis 50 $cm^3$ Lösungsmittel verwendet. Diese Grenzen sind nicht kritisch, es können beispielsweise je 1 g der Iod enthaltenden Verbindung auch mehr als 50 $cm^3$ Lösungsmittel eingesetzt werden, doch genügt es im allgemeinen, eine Lösungsmittelmenge, die in dem genannten Bereich liegt, zu wählen, vorzugsweise werden 1 bis 20 $cm^3$ Lösungsmittel je 1 g der Iod enthaltenden Verbindung angewendet. Geeignete dipolare, aprotische Lösungsmittel sind zum Beispiel Dioxan, Aceton, Carbonsäurealkylester, wie Ethylacetat, Dialkylether von Glykol und Polyglykolen, wie Tetraethylenglykoldimethylether, Dichlormethan und N,N-Dimethylformamid. Besonders gute Ergebnisse werden erhalten, wenn die erfindungsgemäße Umsetzung in mindestens einem der folgenden Lösungsmittel: Tetrahydrofuran, N,N-Dimethylacetamid, Acetonitril, Benzonitril, Nitrobenzol erfolgt. Es können auch Mischungen verschiedener dipolarer, aprotischer Lösungsmittel verwendet werden. Zweckmäßig wird ein solches Lösungsmittel beziehungsweise Lösungsmittelgemisch angewendet, das bei normalem Atmosphärendruck mindestens 10 °C, vorteilhaft mindestens 20 °C, über oder unter der zu erzeugenden, weitgehend fluorierten brom- oder chlorhaltigen Verbindung siedet.

Die oben beschriebene Umsetzung wird bei einer Temperatur von 20 bis 140 °C durchgeführt, wobei sie zweckmäßig unter normalem Atmosphärendruck oder unter dem autogenen Druck der Reaktionsmischung stattfindet. Die Anwendung höherer Drucke ist in der Regel nicht erforderlich und stellt einen unnötigen Aufwand dar. Unterhalb 20 °C verläuft die Reaktion im allgemeinen zu langsam, oberhalb 140 °C wird vermehrt die Bildung unerwünschter Nebenprodukte festgestellt. Vorzugsweise wird bei Temperaturen von 50 bis 90 °C gearbeitet.

Die Reaktionsdauer hängt von der angewendeten Temperatur und den eingesetzten Ausgangsstoffen ab und beträgt im allgemeinen 1 bis 20 Stunden; längere Reaktionsdauern sind möglich, jedoch wird meist kein zusätzlicher Effekt beobachtet, der die immer schlechter werdenden Raum-Zeit-Ausbeuten rechtfertigen würde. Häufig werden bei einer Reaktionsdauer von 2 bis 8 Stunden gute Ergebnisse erhalten.

Die erfindungsgemäße Reaktion wird vorteilhaft unter Ausschluß von Sauerstoff in einem Inertgas, beispielsweise Stickstoff oder Argon, durchgeführt.

Nach Beendigung der Reaktion kann die Mischung auf unterschiedliche Weise aufgearbeitet werden, beispielsweise durch Abdestillieren aller flüchtigen Komponenten einschließlich der Lösungsmittel unter Anwendung von Unterdruck bei Sumpftemperaturen bis zu maximal etwa 100 °C. Das so erhaltene Destillat wird anschließend durch fraktionierte Destillation in die Komponenten (erzeugte, weitgehend fluorierte Chlor beziehungsweise Brom enthaltende Verbindung, nicht umgesetzte Iod enthaltende Verbindung und Lösungsmittel) aufgetrennt, wobei die letzteren beiden wieder eingesetzt werden können. In einer bevorzugten Ausführungsform wird in dem erzeugten Rohdestillat die Menge der weitgehend fluorierten Iod enthaltenden Verbindung analytisch bestimmt und das erfindungsgemäße Verfahren ohne oder mit nur geringer Zugahe von Lösungsmitteln erneut durchgeführt, wonach wiederum alle flüchtigen Bestandteile abdestilliert und nun durch fraktionierte Destillation aufgetrennt werden.

Eine weitere Aufarbeitungsmöglichkeit ist es, das Reaktionsgemisch mit einem Dialkylether, der in den Alkylgruppen 2 bis 8 C-Atome aufweist, beispielsweise Diethylether, oder mit Diallylether, Dichlormethan oder 1,1,2-Trichlor-1,2,2-trifluorethan (F113) zu verdünnen, wobei mindestens das doppelte Volumen als das der eingesetzten, weitgehend fluorierten iodhaltigen Verbindung verwendet wird. Anschließend wird das Gemisch mit Wasser, zweckmäßig bei einer Temperatur von 18 bis 50 °C, vorzugsweise 20 bis 30 °C, gut durchgemischt, die wäßrige Phase von der organischen Phase getrennt, letztere erneut mit Wasser gewaschen, filtriert und getrocknet, sodann entweder sofort fraktioniert destilliert oder, wie oben beschrieben, erneut erfindungsgemäß umgesetzt und anschließend fraktioniert destilliert.

Aus dem bei der Aufarbeitung verbleibenden Rückstand kann das in der Metall-Komplex-Verbindung als Zentralatom enthaltene Metallkation wiedergewonnen und erneut nach Regenerierung der Komplex-Verbindung vor Zugabe oder in situ in der Reaktionsmischung wieder eingesetzt werden.

Die nach dem erfindungsgemäßen Verfahren erzeugten 1 oder 2 Bromatome enthaltenden, weitgehend fluorierten aliphatischen Kohlenwasserstoffe können im medizinischen Sektor eingesetzt werden, zum Beispiel als Kontrastmittel bei Untersuchungen mit Röntgenstrahlen oder mit Ultraschall, beispielsweise zur Sichtbarmachung von Tumoren, zur Organperfusion sowie in wäßriger Emulsion als Blutersatzstoff. Weitere Anwendungen der 1 oder 2 Brom- oder Chloratome enthaltenden, weitgehend fluorierten aliphatischen Kohlenwasserstoffe sind Hochtemperatur-Inertflüssigkeiten und Kontrastmittel für die $^{19}F$-Kernresonanz-Spektral-(NMR)-Analyse und Tomographie.

Wie bereits eingangs erwähnt, ermöglicht es das erfindungsgemäße Verfahren, in wenig aufwendigen Apparaturen ohne Schwierigkeiten bezüglich Korrosion und besondere Sicherheitsvorkehrungen zu arbeiten. Bei dem neuen Verfahren fallen bisweilen in geringeren Mengen Nebenprodukte an, die auf üblichem Wege isoliert werden können und verschiedene Verwendungen haben. Die Verbindungen, die anstelle des Iodatoms ein Wasserstoffatom enthalten, können beispielsweise als Wärmeträgerflüssigkeiten verwendet werden.

Nachfolgende Beispiele sollen die Erfindung näher erläutern.

Die Analysenergebnisse wurden wie folgt gewonnen: Eine Probe wird nach Zugabe von $CDCl_3$ durch $^{19}F$-Kernresonanz untersucht. Für $R_FY$-Verbindungen (Y = I, Br, Cl) unterscheiden sich die chemischen Verschiebungen der Fluoratome in $\alpha$- und $\beta$-Position zu Y in typischer Weise: Im Vergleich zu $R_FI$ sind die $\alpha$-, $\beta$-Resonanzen von $R_FBr$ zu höherem Feld und diejenigen von $R_FCl$ zu noch höherem Feld verschoben. Es wird eine zweite Probe gaschromatographisch untersucht. Dazu wird eine Säule mit 10 % UCCW (ein Polyvinyl-Methylsilicon von Hewlett Packard) auf Embacel ($SiO_2$ mit großer Oberfläche, 60 bis 100 mesh-Partikel) benutzt. Helium dient als Trägergas; es wird ein Wärmeleitfähigkeitsdetektor eingesetzt. Die Zuordnung der gaschromatographischen Peaks wird durch Vergleichsgaschromatographie von Reinsubstanzen abgesichert. Die aus $^{19}F$-NMR und Gaschromatographie erhaltenen Ergebnisse stimmen meist gut überein. Die nachfolgend angegebenen Werte (in Mol-%) sind die Mittelwerte aus NMR- und Gaschromatographiedaten.

Die Beispiele 1 bis 14 werden wie folgt durchgeführt:

Die für die verschiedenen Umsetzungen verwendeten Chemikalien werden zunächst sorgfältig getrocknet. Das eingesetzte Perfluoralkyliodid hat eine Reinheit von mindestens 99,5 %, es wird zur Entfernung etwa vorhandener Spuren freien Halogens mit etwas Kupferpulver behandelt und in brauner Flasche im Dunkeln aufbewahrt.

Ein Vierhalskolben mit 50 cm³ Inhalt, der mit einem Kühler, einem Gaseinleitungsrohr und einem Kontaktthermometer ausgerüstet ist, wird evakuiert und mit einem Hochleistungsfön getrocknet. Nach Aufhebung des Vakuums mit trockenem Argon wird auf dem Kühler ein mit Siliconöl gefülltes Ventil befestigt, in den Kolben ein mit Polytetrafluorethylen beschichteter Magnetrührstab gegeben und der Kolben in ein Heizbad gebracht.

Nun wird die in nachfolgender Tabelle angegebene Lösungsmittelmenge in den Kolben gegeben und mit Argon gesättigt. Anschließend werden unter Rühren die in der Tabelle aufgeführten Reaktionspartner, sofern es sich um feste Stoffe handelt, in feinverteilter Form zugegeben und die Mischung auf die aus der Tabelle ersichtliche Temperatur aufgeheizt und während der angegebenen Zeit auf dieser Temperatur gehalten. Dann wird die Reaktionsmischung auf 20 °C abgekühlt und zunächst 4 cm³ Diethylether und dann 20 cm³ Wasser zugegeben. Während der gesamten Zeit wird der Kolbeninhalt gerührt. Nun wird das Rühren beendet, es scheiden sich eine wäßrige und eine Ether enthaltende Schicht ab. Letztere wird abgetrennt, dreimal mit Wasser ausgeschüttelt, getrocknet und je eine Probe mit $^{19}F$-Kernresonanz-Spektralanalyse sowie gaschromatographisch untersucht. In nachfolgender Tabelle sind die aus beiden Analysenmethoden gemittelten Werte angegeben.

In der Tabelle bedeuten:

I = $(Ph_3P)_2NiBr_2$ Ph = Phenyl
II = $[(C_4H_9)_3P]_2NiBr_2$
III = $[(C_2H_5O)_3P]_2NiBr_2$
IV = $[(PhO)_3P]_2NiBr_2$
V = $(Ph_3P)_2NiCl_2$
VI = $(Ph_3P)_2Rh(CO)Br$
VII = $[Ph_2P(CH_2)_2PPh_2]NiBr_2$
DMA = Dimethylacetamid

1) = + zusätzlich 1 mmol Cetyltrimethylammoniumbromid

2) 49,8 Mol-% $I(CF_2)_4Br$ + 9,5 Mol-% $Br(CF_2)_4Br$

In den Beispielen 1 bis 5 und 9 wird $C_6F_{13}Br$, in den Beispielen 6, 8, 10, 11 und 13 wird $C_8F_{17}Br$, im Beispiel 7 $C_8F_{17}Cl$ und
im Beispiel 14 $CF_3CF_2CF_2OCF(CF_3)CF_2CF_2Br$ erzeugt.

Unter "H enthaltende Verbindung" ist jeweils die Verbindung zu verstehen, die anstelle des im Ausgangsprodukt vorhandenen Iodatoms ein Wasserstoffatom enthält.

TABELLE

| Beispiel Nr. | Iod enthaltende Verbindung a) | Menge a) mmol | $Br^-$ oder $Cl^-$ enthaltendes Salz b) | Menge b) mmol | Metallkomplex c) | Menge c) mmol | Lösungsmittel | Menge $cm^3$ | mol $Br^-$ oder $Cl^-$ je 1 mol Iod in a) | mol c) je 1 mol Iod in a) |
|---|---|---|---|---|---|---|---|---|---|---|
| 1 | $C_6F_{13}I$ | 2 | LiBr | 2 | I | 2 | $CH_3CN$ | 10 | 1 | 1 |
| 2 | $C_6F_{13}I$ | 2 | NaBr | 2 | I | 2 | $CH_3CN$ | 10 | 1 | 1 |
| 3 | $C_6F_{13}I$ | 2 | LiBr | 2 | II | 2 | $CH_3CN$ | 10 | 1 | 1 |
| 4 | $C_6F_{13}I$ | 2 | LiBr | 2 | III | 2 | $CH_3CN$ | 10 | 1 | 1 |
| 5 | $C_6F_{13}I$ | 2 | LiBr | 2 | IV | 2 | $CH_3CN$ | 10 | 1 | 1 |
| 6 | $C_8F_{17}I$ | 2 | LiBr | 2 | II | 2 | DMA | 10 | 1 | 1 |
| 7 | $C_8F_{17}I$ | 2 | LiCl | 2 | V | 2 | $C_6H_5NO_2$ | 10 | 1 | 1 |
| 8 | $C_8F_{17}I$ | 5 | LiBr | 7,5 | I | 0,25 | $CH_3CN$ | 10 | 1,5 | 0,05 |
| 9 | $C_6F_{13}I$ | 5 | LiBr 1) | 5 + 1 | I | 1 | $CH_3CN$ | 10 | 1,2 | 0,2 |
| 10 | $C_8F_{17}I$ | 5 | $MnBr_2$ | 3,75 | I | 0,5 | $CH_3CN$ | 10 | 1,5 | 0,1 |
| 11 | $C_8F_{17}I$ | 5 | $MgBr_2$ | 3,75 | I | 0,5 | $CH_3CN$ | 10 | 1,5 | 0,1 |
| 12 | $I(CF_2)_4I$ | 2 | LiBr | 6 | I | 0,4 | $CH_3CN$ | 20 | 1,5 | 0,1 |
| 13 | $C_8F_{17}I$ | 1 | LiBr | 1 | VI | 1 | $CH_3CN$ | 5 | 1 | 1 |
| 14 | $C_3F_7OCF(CF_3)(CF_2)_2I$ | 5 | LiBr | 7,5 | VII | 0,5 | DMA | 10 | 1,5 | 0,1 |

(Fortsetzung)

TABELLE (Fortsetzung)

| Beispiel Nr. | $cm^3$ Lösungsmittel je 1 g a) | Temperatur °C | Zeit h | Analysen-Mittelwerte Mol-% | | | |
|---|---|---|---|---|---|---|---|
| | | | | Erzeugte Br oder Cl enthaltende Verbindung | Nicht umgesetzte Iod enthaltende Verbindung | H enthaltende Verbindung | Nicht definierter Rest |
| 1 | 11,2 | 80 | 7 | 85,9 | - | - | - |
| 2 | 11,2 | 80 | 7 | 91 | 9 | - | - |
| 3 | 11,2 | 70 | 6 | 83 | 16 | 1 | - |
| 4 | 11,2 | 80 | 7 | 70,7 | 22,1 | 7,2 | - |
| 5 | 11,2 | 80 | 7 | 89,7 | 10,3 | - | - |
| 6 | 9,2 | 60 | 5 | 64 | 31 | 5 | - |
| 7 | 9,2 | 70 | 7 | 78 | 22 | - | - |
| 8 | 3,7 | 80 | 6,5 | 75 | 24,5 | - | 0,5 |
| 9 | 4,5 | 80 | 2 | 47,7 | 51,6 | - | 0,7 |
| 10 | 3,7 | 80 | 7 | 54,5 | 42,5 | - | 3,0 |
| 11 | 3,7 | 80 | 7 | 61,5 | 38,5 | - | - |
| 12 | 22 | 80 | 7 | 2) | 40,7 | - | - |
| 13 | 9,2 | 80 | 7 | 32,6 | 58,6 | - | 8,8 |
| 14 | 3,9 | 60 | 7 | 68 | 30 | 1,5 | 0,5 |

## Patentansprüche

1. Verfahren zur Herstellung von weitgehend fluorierten aliphatischen Kohlenwasserstoffen, die 1 oder 2 Brom- oder Chloratome im Molekül aufweisen aus den entsprechenden weitgehend fluorierten aliphatischen Kohlenwasserstoffen, die 1 oder 2 primär oder sekundär gebundene Iodatome im Molekül aufweisen, durch Umsetzung von 1 mol gebundenem Iodatom in der Iodverbindung mit 1 bis 4 mol Bromid- oder Chloridionen, die als Salze mit mindestens einem der folgenden Kationen: Li, Na, K, Mg, Ca, Mn oder substituiertes Ammonium vorliegen, in mindestens einem dipolaren, aprotischen Lösungsmittel bei 20 bis 140 °C unter normalem Atmosphärendruck oder unter dem autogenen Druck der Reaktionsmischung, dadurch gekennzeichnet, daß die Umsetzung durchgeführt wird in Gegenwart einer Metall-Komplex-Verbindung, die als Zentralatom Chrom, Nickel, Kobalt oder Rhodium enthält und die je Zentralatom bei der Herstellung der weitgehend fluorierten aliphatischen Bromide 1, 2 oder 3 Bromatome, bei der Herstellung der weitgehend fluorierten aliphatischen Chloride 1, 2 oder 3 Chloratome und als weitere Liganden mindestens einen der folgenden Formeln enthält:

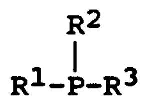

worin bedeuten: $R^1$, $R^2$, $R^3$, gleich oder verschieden, Alkyl mit 1 bis 8 C-Atomen, Alkoxy mit 1 bis 8 C-Atomen, Phenyl oder Phenoxy, das mit Alkyl-, Alkoxy-, Phenyl- oder Phenoxy-Gruppen substituiert sein kann und das 6 bis 14 C-Atome aufweist, oder

$$(Phenyl)_2P\text{-}R^4\text{-}P(Phenyl)_2$$

worin $R^4$ einen Alkylenrest mit 1 bis 6 C-Atomen bedeutet.

2. Verfahren nach Anspruch 1, dadurch gekennzeichnet, daß Perfluoralkyliodide mit 2 bis 12 C-Atomen für die Umsetzung zur Herstellung der Perfluoralkylbromide oder der Perfluoralkylchloride eingesetzt werden.

3. Verfahren nach Anspruch 1 oder 2, dadurch gekennzeichnet, daß die Umsetzung in Gegenwart einer Metall-Komplex-Verbindung des Nickels durchgeführt wird, die bei der Herstellung der weitgehend fluorierten aliphatischen Bromide 2 Bromatome oder bei der Herstellung der weitgehend fluorierten aliphatischen Chloride 2 Chloratome aufweist.

4. Verfahren nach einem oder mehreren der Ansprüche 1 bis 3, dadurch gekennzeichnet, daß die Umsetzung in mindestens einem der folgenden Lösungsmittel: Tetrahydrofuran, N,N-Dimethylacetamid, Acetonitril, Benzonitril, Nitrobenzol erfolgt.

5. Verfahren nach einem oder mehreren der Ansprüche 1 bis 4, dadurch gekennzeichnet, daß die Umsetzung bei 50 bis 90 °C durchgeführt wird.

6. Verfahren nach einem oder mehreren der Ansprüche 1 bis 5, dadurch gekennzeichnet, daß die Bromidionen oder die Chloridionen als Lithiumsalze vorliegen.

## Claims

1. A process for the preparation of extensively fluorinated aliphatic hydrocarbons having 1 or 2 bromine or chlorine atoms in the molecule, from the corresponding extensively fluorinated aliphatic hydrocarbons having 1 or 2 primary or secondary iodine atoms in the molecule, by reacting 1 mol of bound iodine atom in the iodine compound with 1 to 4 mol of bromide or chloride ions present as salts with at least one of the following cations: Li, Na, K, Mg, Ca, Mn or substituted ammonium, in at least one dipolar aprotic solvent at 20 to 140°C under normal atmospheric pressure or under the autogenous pressure of the reaction mixture, which comprises carrying out the reaction in the presence of a metal complex compound which contains chromium, nickel, cobalt or rhodium as the central atom and contains, per central atom, 1, 2 or 3 bromine atoms in the case of the preparation of the extensively fluorinated aliphatic bromides, or 1, 2 or 3 chlorine atoms in the case of the preparation of the extensively fluorinated aliphatic chlorides, and, as additional ligands, at least one ligand of the following formulae:

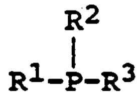

wherein $R^1$, $R^2$ and $R^3$, which are identical or different, are alkyl having 1 to 8 carbon atoms, alkoxy having 1 to 8 carbon atoms, phenyl or phenoxy, which can be substituted by alkyl, alkoxy, phenyl or phenoxy groups and has 6 to 14 carbon atoms, or

$$(phenyl)_2P\text{-}R^4\text{-}P(phenyl)_2$$

wherein $R^4$ is an alkylene radical having 1 to 6 carbon atoms.

2. A process as claimed in claim 1, wherein perfluoroalkyl iodides having 2 to 12 carbon atoms are used for the reaction to prepare the perfluoroalkyl bromides or the perfluoroalkyl chlorides.

3. A process as claimed in claim 1 or 2, wherein the reaction is carried out in the presence of a metal complex compound of nickel which has 2 bromine atoms in the case of the preparation of the extensively fluorinated aliphatic bromides, or 2 chlorine atoms in the case of the preparation of the extensively fluorinated aliphatic chlorides.

4. A process as claimed in one or more of claims 1 to 3, wherein the reaction is carried out in at least one of the following solvents: tetrahydrofuran, N,N-dimethylacetamide, acetonitrile, benzonitrile and nitrobenzene.

5. A process as claimed in one or more of claims 1 to 4, wherein the reaction is carried out at 50 to 90°C.

6. A process as claimed in one or more of claims 1 to 5, wherein the bromide ions or the chloride ions are present as lithium salts.

**Revendications**

1. Procédé de préparation d'hydrocarbures aliphatiques fortement fluorés, qui présentent dans leur molécule un ou deux atomes de brome ou de chlore, obtenus à partir d'hydrocarbures aliphatiques largement fluorés correspondants, ayant dans leur molécule 1 ou 2 atomes d'iode à liaison primaire ou secondaire, par réaction d'une mole d'atome d'iode fixé dans le composé de l'iode avec 1 à 4 moles d'ions bromure ou chlorure, qui sont présents sous forme de sels avec au moins l'un des cations suivants : Li, Na, K, Mg, Ca, Mn ou un groupe ammonium substitué, dans au moins un solvant aprotique dipolaire, entre 20 et 140°C sous la pression atmosphérique normale ou sous la pression autogène du mélange réactionnel, procédé caractérisé en ce qu'on effectue la réaction en présence d'un composé complexe de métal, qui contient comme atome central du chrome, du nickel, du cobalt ou rhodium et qui, pour chaque atome central contient, lors de la préparation des bromures aliphatiques fortement fluorés 1, 2 ou 3 atomes de brome, lors de la préparation de chlorures aliphatiques fortement fluorés 1, 2 ou 3 de chlore, et, comme autres ligands, au moins un ligand répondant à la formule suivante :

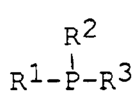

formule dans laquelle $R^1$, $R^2$, $R^3$, identiques ou différents, représentent chacun un groupe alkyle ayant 1 à 8 atomes de carbone, alcoxy ayant 1 à 8 atomes de carbone, phényle ou phénoxy, pouvant être substitué par des groupes alkyle, alcoxy, phényle ou phénoxy et présentant 6 à 14 atomes de carbone, ou de formule :

$$(phényl)_2P\text{-}R^4(phényl)_2$$

dans laquelle $R^4$ représente un reste alkylène ayant 1 à 6 atomes de carbone.

2. Procédé selon la revendication 1, caractérisé en ce qu'on utilise des iodures de perfluoro-alkyles comportant 2 à 12 atomes de carbone, pour la réaction de préparation des bromures de perfluoro-alkyles ou chlorures de perfluoro-alkyles.

3. Procédé selon la revendication 1 ou 2, caractérisé en ce qu'on effectue la réaction en présence d'un composé complexe de métal , qui est un composé de nickel présentant 2 atomes de brome en cas de préparation de bromures aliphatiques fortement fluorés, ou présentant 2 atomes de chlore dans le cas de préparation de chlorures aliphatiques fortement fluorés.

4. Procédé selon une ou plusieurs des revendications 1 à 3, caractérisé en ce que la réaction a lieu dans au moins l'un des solvants suivants: le tétrahydrofuranne, le N,N-diméthylacétamide, l'acétonitrile, le benzonitrile, le nitrobenzène.

5. Procédé selon une ou plusieurs des revendications 1 à 4, caractérisé en ce qu'on conduit la réaction entre 50 et 90°C.

6. Procédé selon l'une ou plusieurs des revendications 1 à 5, caractérisé en ce que les ions bromures ou les ions chlorures sont présents sous forme de sels de lithium.